# EUROPEAN PATENT APPLICATION

(11) **EP 3 345 622 A1**
(43) Date of publication of application: **11.07.2018**
(21) Application number: 16841886.1
(22) Date of filing: 31.08.2016
(51) Int. Cl.: A61K 45/00, A61K 31/551, A61P 27/02, A61P 27/06, A61P 43/00

(54) **AGENT FOR ENHANCING OCULAR HYPOTENSIVE EFFECT**

(30) Priority: 31.08.2015 JP 2015170673
(71) Applicant: Kowa Company, Ltd., Nagoya-shi, Aichi 460-8625 (JP)
(72) Inventor: KAWAMATA, Kenji, Tokyo 103-0023 (JP); MARUO, Kazushi, Tokyo 103-8433 (JP); SUGANAMI, Hideki, Tokyo 103-8433 (JP)
(74) Representative: Adam, Holger
(86) International application number: PCT/JP2016/075426
(87) International publication number: WO 2017/038856

(57) **Abstract**

The present invention pertains to a drug available for long-term continuous administration for preventing or treating glaucoma or ocular hypertension, said drug showing no decrease in efficacy during long-term administration and exhibiting a stable ocular hypotensive effect. The present invention pertains to a therapeutic agent for glaucoma or ocular hypertension that is to be continuously administered for a long period of time, said drug comprising an Rho kinase inhibitor, more specifically, to a therapeutic agent comprising an Rho kinase inhibitor for treating glaucoma or ocular hypertension in a patient suffering from glaucoma or ocular hypertension who has been administered with a therapeutic agent comprising the Rho kinase inhibitor for treating glaucoma or ocular hypertension continuously over a definite period of time and needs a further enhanced ocular hypotensive effect.

## Description

### Technical Field

The present invention relates to a drug available for long-term continuous administration for preventing or treating glaucoma or ocular hypertension, and further relates to a novel enhanced agent for intra-ocular pressure reduction over a long duration.

### Background Art

Glaucoma is a disease characterized by functional structural abnormality of the eye, causing a characteristic change in the optic nerve and visual field and its optic nerve disorder can usually be improved or suppressed by sufficiently reducing an intra-ocular pressure. A disease type lacking a characteristic morphological change of the optic nerve and the presence of visual field abnormality is called ocular hypertension.

Glaucoma is classified into three types of primary glaucoma, secondary glaucoma, and developmental glaucoma. Primary glaucoma is a generic name of glaucoma which is unclear in cause, is common in middle-aged and elderly people, and is the most common type in glaucoma. There are also many patients who develop normal tension glaucoma without elevation of an intra-ocular pressure. Secondary glaucoma is glaucoma caused by an obvious cause such as inflammation or injuries, and is developed because of eyes, such as uveitis or eye injuries, and is also developed by neovascularization due to diabetes, long-term use of steroid hormones for treatment of other diseases, and the like. Each of primary glaucoma and secondary glaucoma is classified into two types of open angle glaucoma and angle closure glaucoma depending on how a flow of aqueous humor is hindered. Note that the angle has a structure including a transition portion of the corneal sclera and a root portion of the iris. The closed angle refers to a state in which a part or the entire of the angle is closed, and the open angle refers to a state in which the angle is open. Developmental glaucoma is glaucoma caused by inherently inadequate growth in the angle and obstruction of aqueous humor outflow. The only reliable therapy based on evidence for glaucoma treatment, including normal tension glaucoma, is to lower an intra-ocular pressure.

Drug therapy is the first choice for use in a treatment of glaucoma, however, in a case where an intra-ocular pressure cannot be controlled with a drug or a patient suffering from angle closure glaucoma is having an acute glaucoma attack, laser therapy (laser trabeculoplasty), surgical therapy (trabeculectomy and trabeculotomy), or the like is performed. In drug therapy for glaucoma, sympathomimetic drugs (nonselective stimulants such as epinephrine, and α2 stimulants such as apraclonidine and brimonidine), sympatholytic drugs (β-blockers such as timolol, befunolol, carteolol, nipradilol, betaxol, levobunolol, and metipranolol, and α1-blockers such as bunazosin hydrochloride), parasympathomimetic drugs (pilocarpine or the like), carbonic anhydrase inhibitors (acetazolamide, brinzolamide, dorzolamide, and the like), prostaglandins (PGs) (isopropyl unoprostone, latanoprost, travoprost, bimatoprost, tafluprost, and the like), Rho kinase inhibitors (ripasudil or the like), and the like are used.

Among these drugs, Y-27632 known as a Rho kinase inhibitor lowers an intra-ocular pressure by promoting aqueous humor outflow from an outflow path of the trabecular meshwork at an angle portion (Non-Patent Document 1). Furthermore, it is suggested that an action thereof is caused by cytoskeletal change in trabecular meshwork cells (Non-Patent Documents 1 and 2).

A therapeutic agent for glaucoma is administered over a long duration, and therefore is appropriately selected and changed from the above drugs in view of an intra-ocular pressure-lowering effect, a side effect, and the like. A β-blocker is used as a first-line drug in addition to a PG. In a case of particularly commonly used timolol, a phenomenon to attenuate an intra-ocular pressure-lowering effect by long-term administration (long term drift) is known (Non-Patent Documents 3 and 4). It is considered that an action of timolol is restored by providing a rest period of timolol with a substitute (Non-Patent Document 3), but long-term continuous administration of timolol often causes a problem.

### Citation List

### Non-Patent Document

Non-Patent Document 1: IOVS, 42 (1), 137-144 (2001)
Non-Patent Document 2: IOVS, 42 (5), 1029-1037 (2001)
Non-Patent Document 3: IOVS, 31 (2), 354-358 (1990)
Non-Patent Document 4: Surv Ophthalmol, 28, 235-240 (1983)

### Summary of the Invention

### Problems to be Solved by the Invention

The present invention relates to providing a drug available for long-term continuous administration for preventing or treating glaucoma or ocular hypertension, the drug exhibiting no decrease in efficacy during long-term administration and exhibiting a stable intra-ocular pressure-lowering effect.

### Means for Solving the Problems

As a result of intensive studies to solve the above problems, the present inventors have found the following fact surprisingly. That is, by long-term administration of a 0.4% Glanatec ® ophthalmic solution containing a Rho kinase inhibitor ripasudil (general name, chemical name: (S)-(-)-1-(4-fluoro-5-isoquinolinesulfonyl)-2-methyl-1,4-homopiperazine) as an active ingredient, no decrease in an intra-ocular pressure-lowering effect is observed. In addition, by continuous administration for 26 weeks or more, an intra-ocular pressure-lowering effect is further enhanced. The present inventors have confirmed that the Rho kinase inhibitor is a drug available for long-term continuous administration for preventing or treating glaucoma or ocular hypertension, and have further found an intra-ocular pressure-lowering effect by a novel action mechanism different from conventional short-term administration by long-term continuous administration of the Rho kinase inhibitor, thus completing the present invention.

That is, the present invention relates to a Rho kinase inhibitor-containing therapeutic agent available for long-term continuous administration for treating glaucoma or ocular hypertension. The present invention also relates to a Rho kinase inhibitor-containing therapeutic agent for treating glaucoma or ocular hypertension in a patient suffering from glaucoma or ocular hypertension who has been administered with a Rho kinase inhibitor-containing therapeutic agent for treating glaucoma or ocular hypertension continuously over a definite period of time and needs a further enhanced intra-ocular pressure-lowering effect.

If the present invention is described in more detail, the present invention relates to the following invention.
(1) A therapeutic agent for glaucoma or ocular hypertension for long-term continuous administration, comprising a Rho kinase inhibitor.
(2) The therapeutic agent according to (1), wherein glaucoma or ocular hypertension is glaucoma or ocular hypertension with a baseline intra-ocular pressure of 15 mmHg or higher, 18 mmHg or higher, or 21 mmHg or higher at the time before treatment.
(3) The therapeutic agent according to (1) or (2), wherein the long-term continuous administration is continuous administration for 26 weeks or more or 28 weeks or more.
(4) An agent for improving and enhancing aqueous humor outflow resistance, comprising a Rho kinase inhibitor.
(5) The agent for improving and enhancing of aqueous humor outflow resistance according to (4), wherein the agent for improving and enhancing of aqueous humor outflow resistance is achieved by long-term continuous administration.
(6) The agent for improving and enhancing of aqueous humor outflow resistance according to (5), wherein the long-term continuous administration is continuous administration for 26 weeks or more or 28 weeks or more.
(7) The agent for improving and enhancing of aqueous humor outflow resistance according to any one of (4) to (6), wherein the agent is for a patient having aqueous humor outflow resistance suffers from glaucoma or ocular hypertension with a baseline intra-ocular pressure of 15 mmHg or higher, 18 mmHg or higher, or 21 mmHg or higher at the time before treatment.
(8) A trabecular meshwork function adjusting agent comprising a Rho kinase inhibitor.
(9) The trabecular meshwork function adjusting agent according to (8), wherein the trabecular meshwork function adjusting agent for long-term continuous administration.
(10) The trabecular meshwork function adjusting agent according to (9), wherein the long-term continuous administration is continuous administration for 26 weeks or more or 28 weeks or more.
(11) The trabecular meshwork function adjusting agent according to any one of (8) to (11), wherein the trabecular meshwork function adjusting agent is a trabecular meshwork function adjusting agent for a patient with an baseline intra-ocular pressure of 15 mmHg or higher, 18 mmHg or higher, or 21 mmHg or higher at the time before treatment.
(12) A therapeutic agent for glaucoma or ocular hypertension, comprising a Rho kinase inhibitor as an active ingredient, wherein the therapeutic agent is for use in the treatment of glaucoma or ocular hypertension in a patient who has been administered with a Rho kinase inhibitor-containing therapeutic agent for glaucoma or ocular hypertension over a definite period and is suffering from glaucoma or ocular hypertension considered to require a further enhanced intra-ocular pressure-lowering action.
(13) The therapeutic agent according to (12), wherein the definite period for administration is 15 weeks or more, 20 weeks or more, 26 weeks or more, or 28 weeks or more.
(14) The therapeutic agent according to (12) or (13), wherein the glaucoma or ocular hypertension considered to require a further enhanced intra-ocular pressure-lowering action is a glaucoma or ocular hypertension with a baseline intra-ocular pressure of 15 mmHg or higher, 18 mmHg or higher, or 21 mmHg or higher at the time before treatment.
(15) The therapeutic agent according to any one of (12) to (14), wherein the glaucoma or ocular hypertension considered to require a further enhanced intra-ocular pressure-lowering action is open angle glaucoma.
(16) The agent according to any one of (1) to (15), wherein the Rho kinase inhibitor is ripasudil.
(17) The agent according to any one of (1) to (16), wherein the Rho kinase inhibitor-containing therapeutic agent administered continuously for long-term or administered after a definite period is an agent for monotherapy.

### Effects of the Invention

The present invention can provide a drug for preventing or treating glaucoma or ocular hypertension, the drug exhibiting no decrease in efficacy during long-term administration and exhibiting an enhanced intra-ocular pressure-lowering effect.

The therapeutic agent of the present invention does not need to provide a rest period and can be administered continuously for a long duration. In addition, the therapeutic agent can obtain a stable intra-ocular pressure-lowering effect over a long duration. Furthermore, the therapeutic agent can achieve an enhanced intra-ocular pressure-lowering effect in a second half of an administration period, for example, after 26 weeks or 29 weeks, and is effective for treating glaucoma such as open angle glaucoma or ocular hypertension, requiring a further enhanced intra-ocular pressure-lowering effect over a long duration.

In addition, the therapeutic agent of the present invention exhibits remarkable efficacy even by monotherapy, but by additive therapy in combination with a therapeutic agent comprising a PG or a therapeutic agent comprising a β-blocker.

### Brief Description of Drawings

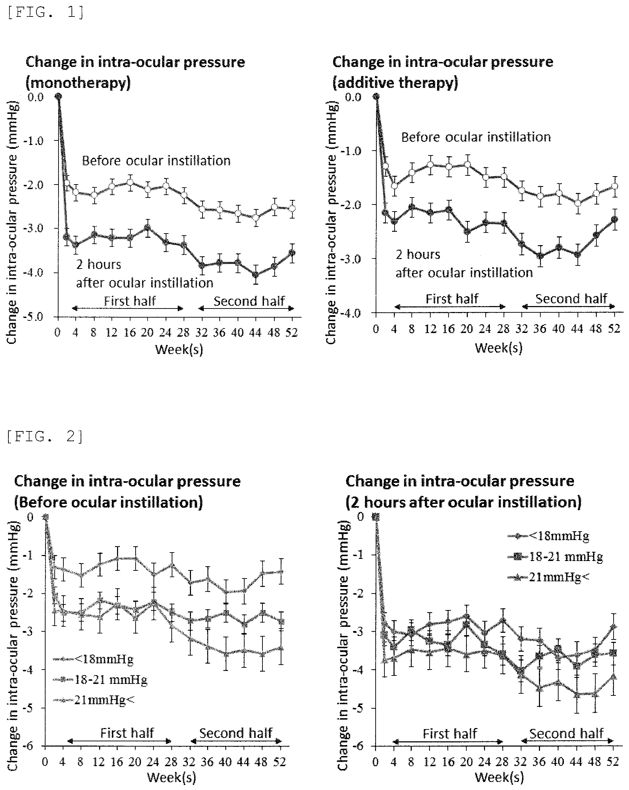

A left side of FIG. 1 illustrates the amount of change in intra-ocular pressure over time at every 4 weeks (mmHg, least mean square value ± SE) in a cohort for monotherapy of a Rho kinase inhibitor-containing therapeutic agent of the present invention (0.4% Glanatec ® ophthalmic solution), in which an open circle (light blue circle in the original drawing) indicates an intra-ocular pressure before ocular instillation (immediately before ocular instillation in the morning), and a filled circle (red circle in the original drawing) indicates an intra-ocular pressure at 2 hours after ocular instillation. A right side of FIG. 1 illustrates the amount of change in an intra-ocular pressure over time at every 4 weeks (mmHg, least mean square value ± SE) in a cohort for combination therapy (combination group of a therapeutic agent comprising a PG, a β-blocker, or a PG and a β-blocker, and a 0.4% Glanatec ® ophthalmic solution), in which an open circle (light blue circle in the original drawing) indicates an intra-ocular pressure before ocular instillation, and a filled circle (red in the original drawing) indicates an intra-ocular pressure at 2 hours after ocular instillation.

A left side of FIG. 2 illustrates the amount of change in intra-ocular pressure before ocular instillation over time at every 4 weeks (mmHg, least mean square value ± SE) in a cohort for monotherapy of the Rho kinase inhibitor-containing therapeutic agent of the present invention (0.4% Glanatec ® ophthalmic solution). A left side of FIG. 2 illustrates a patient with a baseline intra-ocular pressure of lower than 18 mmHg before start of treatment (filled diamond (light blue diamond in the original drawing)), a patient with a baseline intra-ocular pressure of 18 mmHg or higher and lower than 21 mmHg (filled square (red square in the original drawing)), and a patient with a baseline intra-ocular pressure of 21 mmHg or higher (filled triangle (light green triangle in the original drawing)). The right side of FIG. 2 illustrates the amount of change in intra-ocular pressure at 2 hours after ocular instillation over time at every 4 weeks (mmHg, least mean square value ± SE) in a cohort for monotherapy of the Rho kinase inhibitor-containing therapeutic agent of the present invention (0.4% Glanatec ® ophthalmic solution). A right side of FIG. 2 illustrates a patient with a baseline intra-ocular pressure of lower than 18 mmHg before start of treatment (filled diamond (light blue diamond in the original drawing)), a patient with a baseline intra-ocular pressure of 18 mmHg or higher and lower than 21 mmHg (filled square (red square in the original drawing)), and a patient with a baseline intra-ocular pressure of 21 mmHg or higher (filled triangle (light green triangle in the original drawing)).

### Modes for Carrying Out the Invention

Examples of a Rho kinase inhibitor used in a therapeutic agent of the present invention include a substance having inhibitory activity on Rho kinase. Examples of such a Rho kinase inhibitor include compounds represented by the following general names and code numbers. Examples thereof include compounds such as ripasudil (chemical name: (S)-(-)-1-(4-fluoro-5-isoquinolinesulfonyl)-2-methyl-1,4-homopiperazine; trade name: 0.4% Glanatec (trademark) ophthalmic solution), AR-13324 (chemical name: 4-[(2S)-3-amino-1-(6-isoquinolinylamino)-1-oxo-2-propanyl] benzyl 2,4-dimethylbenzoate; trade name: Rhopressa (trademark)), AR-12286 (chemical name: (2RS)-2-(dimethylamino)-N-(1-oxo-1,2-dihydroisoquinolin-6-yl)-2-(thiophen-3-yl) acetamide), AS 1892802 (chemical name: N-[(1S)-2-hydroxy-1-phenylethyl]-N'-[4-(4-pyridinyl) phenyl]-urea), DL 0805 (chemical name: 5-nitro-1(2)H-indazole-3-carbonitrile), DW 1865 (chemical name: 2-(1H-indazol-5-yl) amino-4-methoxy-6-piperazinotriazine), Fasudil (chemical name: 1-(5-isoquinolinesulfonyl) homopiperazine), GSK-269962A (chemical name: [N-(3-{[2-(4-amino-1,2,5-oxadiazol-3-yl)-1-ethyl-lH-imidazo [4,5-c]pyridin-6-yl]oxy}phenyl)-4-[2-(4-morpholinyl) ethyl]oxy}benzamide]), GSK-429286A (chemical name: N-(6-fluoro-1H-indazol-5-yl)-2-methyl-6-oxo-4-[4-(trifluoromethyl) phenyl]-1,4,5,6-tetrahydro-3-pyridinecarboxamide), H-1152 (chemical name: 5-[[(2S)-hexahydro-2-methyl-1H-1,4-diazepin-1-yl] sulfonyl]-4-methyl-isoquinoline), KD025 (SLx-2119) (chemical name: 2-(3-(4-((1H-indazol-5-yl) amino) quinazolin-2-yl) phenoxy)-N-isopropylacetamide), PT-262 (chemical name: 7-chloro-6-piperidin-1-yl-quinoline-5,8-dione), RKI-1447 (chemical name: 1-[(3-hydroxyphenyl) methyl]-3-(4-pyridin-4-yl-1,3-thiazol-2-yl) urea), SAR 407899 (chemical name: 6-(piperidin-4-yloxy) isoquinolin-1(2H)-one), SB-772077B (chemical name: (3S)-1-[[2-(4-amino-1,2,5-oxadiazol-3-yl)-1-ethyl-1H-imidazo [4,5-c] pyridin-7-yl] carbonyl]-3-pyrrolidinamine dihydrochloride), SNJ-1656 (Y-39983) (chemical name: (R)-(+)-N-(1H-pyrrolo [2,3-b] pyridin-4-yl)-4-(1-aminoethyl) benzamide), Wf-536 (chemical name: (+)-(R)-4-(1-aminoethyl)-N-(4-pyridyl) benzamide), Y-26732(chemical name: (R)-(+)-trans-N-(4-pyridyl)-4-(1-aminoethyl)-cyclohexanecarboxamide), AMA 0076, BA-1049, BF-66851, BF-66852, BF-66853, KI-23095, AR-13503, AR-13534, INS-117548, LX 7101, (1-benzylpyrrolidin-3-yl)-(1H-indazol-5-yl) amine, (1-benzylpiperidin-4-yl)-(1H-indazol-5-yl) amine, N-[2-(4-fluorophenyl)-6,7-dimethoxy-4-quinazolinyl]-N-(1H-indazol-5-yl) amine, N-4-(1H-indazol-5-yl)-6,7-dimethoxy-N-2-pyridin-4-yl-quinazoline-2,4-diamine, and 4-methyl-5-(2-methyl-[1,4] diazepane-1-sulfonyl) isoquinoline, optical isomers thereof, salts thereof, and solvates thereof. Examples thereof further include compounds disclosed in the following Patent Documents: US 4,678,783, JP 3421217 B2, WO 95/28387 A, WO 97/23222 A, WO 98/06433 A, WO 99/20620 A, WO 99/61403 A, WO 00/09162 A, WO 01/56988 A, WO 02/076976 A, WO 02/076977 A, WO 02/083175 A, WO 02/100833 A, WO 03/059913 A, WO 03/062225 A, WO 03/062227 A, WO 2004/009555 A, WO 2004/022541 A, WO 2004/039796 A, WO 2004/108724 A, WO 2005/003101 A, WO 2005/034866 A, WO 2005/037197 A, WO 2005/037198 A, WO 2005/035501 A, WO 2005/035503 A, WO 2005/035506 A, WO 2005/039564 A, WO 2005/080394 A, WO 2005/103050 A, WO 2006/057270 A, and WO 2007/026664 A, optical isomers thereof, salts thereof, and solvates thereof. Examples thereof further include an antisense nucleic acid against ROCK, an RNA interference inducible nucleic acid (for example, siRNA), a dominant negative mutant, and an expression vector for the nucleic acid.

A preferable Rho kinase inhibitor among these Rho kinase inhibitors is ripasudil, Fasudil, AR-13324, SNJ-1656 (Y-39983), or Y-26732, and a more preferable Rho kinase inhibitor is ripasudil.

In the following description, ripasudil which is one of representative compounds of the Rho kinase inhibitor will be exemplified.

Ripasudil ((S)-(-)-1-(4-fluoro-5-isoquinolinesulfonyl)-2-methyl-1,4-homopiperazine) is a compound having a substance P antagonistic action, a leukotriene D4 antagonistic action, and a Rho kinase inhibitory action. A drug comprising ripasudil as an active ingredient is manufactured and sold as a therapeutic agent for glaucoma and ocular hypertension. Note that ripasudil can be manufactured by a known method, for example, a method described in WO 99/20620 A.

Example of a salt of ripasudil include a salt of an inorganic acid, such as hydrochloric acid, sulfuric acid, nitric acid, hydrofluoric acid, or hydrobromic acid, and a salt of an organic acid, such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid, or camphorsulfonic acid. A hydrochloride is particularly preferable.

Ripasudil or a salt thereof can exist not only as an unsolvated form but also as a hydrate or a solvate. A hydrate is preferable. However, the present invention includes all crystal forms and hydrates or solvates.

Examples of glaucoma include primary glaucoma, secondary glaucoma, and developmental glaucoma.

Examples of primary glaucoma include primary open angle glaucoma in a wide sense such as primary open angle glaucoma or normal tension glaucoma, primary angle closure glaucoma such as primary angle closure glaucoma or plateau iridial glaucoma, and mixed glaucoma which is a combination of primary open angle glaucoma and primary angle closure glaucoma.

Examples of secondary glaucoma include secondary open angle glaucoma and secondary angle closure glaucoma.

Examples of secondary open angle glaucoma include secondary open angle glaucoma with representative pathology of aqueous humor outflow resistance between the trabecular meshwork and the anterior chamber, such as neovascular glaucoma, glaucoma due to heterochromic iridocyclitis, or glaucoma due to intraventricular epithelial proliferation, secondary open angle glaucoma with representative pathology of aqueous humor outflow resistance in the trabecular meshwork, such as steroid glaucoma, exfoliation glaucoma, glaucoma associated with an inflammatory disease (Posner-Schlossman syndrome, sarcoidosis, Behcet's disease, herpetic corneal uveitis, bacterial/fungal endophthalmiti, or the like), glaucoma associated with primary amyloidosis, glaucoma due to uveitis, glaucoma caused by the lens (lens melting glaucoma), glaucoma due to trauma, glaucoma after vitrectic surgery, ghost cell glaucoma, glaucoma after cataract surgery, glaucoma after corneal transplantation, glaucoma due to intra-ocular foreign matters, glaucoma due to intra-ocular tumor, Schwartz syndrome, pigmented glaucoma, or pigment spread syndrome, secondary open angle glaucoma with a representative pathology of aqueous humor outflow resistance behind the Schlemm tube, such as glaucoma associated with eyeball protrusion (such as thyroid ophthalmopathy) or glaucoma due to hyperopia venous hypertension (such as internal carotid arteriovenous fistula), and secondary open angle glaucoma due to supersecretion of aqueous humor.

Examples of secondary angle closure glaucoma include secondary angle closure glaucoma due to pupillary block caused by bulging lens, lens dislocation, lens ophthalmia, or iris cylinder due to posterior synechia of the iris due to uveitis, secondary angle closure glaucoma due to forward movement of tissues located behind the lens, caused by direct closure due to forward movement of iris-lens irrespective of pupillary block caused by bulging lens or lens dislocation, lens ophthalmia, after panretinal photocoagulation, after scleral shortening, intra-ocular tumor, posterior scleritis, uveitis, ciliary choroid delamination due to Harada disease, malignant glaucoma, intra-ocular filling substances, mass vitreous hemorrhage, or retinopathy of prematurity, and secondary angle closure glaucoma due to peripheral anterior synechia of the iris caused irrespective of the anterior chamber depth, caused by uveitis, after corneal transplantation, neovascular glaucoma, iris corneal endothelium (ICE) syndrome, anterior chamber epithelial proliferation, or iris separation.

Examples of developmental glaucoma include, in addition to onset developmental glaucoma and late-onset developmental glaucoma, developmental glaucoma accompanied by other congenital anomaly, such as iridia, Sturge-Weber syndrome, Axenfeld-Rieger syndrome, Peter's anomaly, Marfan syndrome, Weill-Marchesani syndrome, homocystinuria, neurofibromatosis, rubella syndrome, Pierre Robin syndrome, primary vitrector hyperplasia remnant, congenital small cornea, Lowe syndrome, Rubinstein-Taybi syndrome, Hallermann-Streiff syndrome, or congenital varine deformity.

Glaucoma with a high baseline intra-ocular pressure in the present invention means glaucoma with a baseline intra-ocular pressure of 15 mmHg or higher, preferably 18 mmHg or higher, particularly preferably 21 mmHg or higher before start of treatment. Incidentally, an upper limit value of the intra-ocular pressure is not particularly limited, but it goes without saying that the upper limit value is an intra-ocular pressure that can maintain the form of the eyeball.

The term "ocular hypertension" as used in the present invention is also called ophthalmic hypertension, and refers to a symptom exhibiting an abnormally high intra-ocular pressure although no clear lesion is observed in the optic nerve, and includes many ocular hypertensive conditions such as exhibition of a high intra-ocular pressure after surgery. As a preferable baseline intra-ocular pressure of ocular hypertension in the present invention, a baseline intra-ocular pressure before start of treatment is 21 mmHg or higher. Incidentally, an upper limit value of the intra-ocular pressure is not particularly limited, but it goes without saying that the upper limit value is an intra-ocular pressure that can maintain the form of the eyeball.

An action mechanism of the Rho kinase inhibitor-containing therapeutic agent in the present invention is not necessarily clear. However, considering that an intra-ocular pressure-lowering effect is sustained even in long-term continuous administration but also the intra-ocular pressure-lowering effect is enhanced, it is presumed that not only the intra-ocular pressure-lowering effect known in conventional treatment for glaucoma or ocular hypertension but also a novel different action mechanism by long-term administration is involved. As such an action mechanism, it is considered that the trabecular meshwork present near the Schlemm tube at the angle portion may be involved.

An intra-ocular pressure largely depends on increase and decrease of ocular aqueous humor. The aqueous humor is produced in epithelial cells at the ciliary process, exits to the posterior chamber, passes through the pupil margin, and enters the anterior chamber. A part thereof also flows out of a uvea scleral outflow path, but flows mainly out of the eye from the Schlemm tube at the angle portion. The aqueous humor enters the Schlemm tube through the trabecular meshwork. However, the trabecular meshwork has aqueous humor outflow resistance, and it is said that aqueous humor outflow resistance is a source of forming an intra-ocular pressure.

It is presumed that the Rho kinase inhibitor-containing therapeutic agent to be administered continuously for long-term according to the present invention acts on the trabecular meshwork or subsequent tissues by a novel action mechanism, affects aqueous humor outflow resistance, and thereby lowers an intra-ocular pressure. Such a mechanism is not necessarily completely elucidated at the present stage. However, it is considered that a change in the trabecular meshwork due to continuous administration of the Rho kinase inhibitor-containing therapeutic agent of the present invention over a long-term is involved. It is considered that this change may be more remarkably exhibited by continuous administration for 15 weeks or more, 20 weeks or more, 26 weeks or more, or 28 weeks or more, preferably 26 weeks or more or 28 weeks or more. As a result, it is considered that further continuous administration of the Rho kinase inhibitor-containing therapeutic agent of the present invention further enhances an intra-ocular pressure-lowering effect, and makes the intra-ocular pressure-lowering effect more remarkable.

Therefore, it is considered that the Rho kinase inhibitor-containing therapeutic agent for long-term continuous administration according to the present invention promotes some alteration in the trabecular meshwork located in the Schlemm tube or in the vicinity thereof, and that the Rho kinase inhibitor-containing therapeutic agent is particularly effective for treatment of ocular hypertension or glaucoma caused by inhibition of aqueous humor outflow. Therefore, it is considered that the Rho kinase inhibitor-containing therapeutic agent is particularly effective for treatment of open angle glaucoma with representative pathology of aqueous humor outflow resistance in the trabecular meshwork. As for open angle glaucoma, it is considered that the Rho kinase inhibitor-containing therapeutic agent is effective not only for primary open angle glaucoma (wide sense) but also for secondary open angle glaucoma, particularly for secondary open angle glaucoma in which the trabecular meshwork is involved.

As described above, the present invention provides not only a Rho kinase inhibitor-containing therapeutic agent for glaucoma or ocular hypertension for long-term continuous administration, but also provides a novel therapeutic agent for primary open angle glaucoma (wide sense) or secondary open angle glaucoma, particularly for primary open angle glaucoma (wide sense) or secondary open angle glaucoma in which the trabecular meshwork is involved, and ocular hypertension in which the trabecular meshwork is involved.

Therefore, it can be said that the present invention is a Rho kinase inhibitor-containing therapeutic agent for long-term continuous administration for treating primary open angle glaucoma (wide sense) or secondary open angle glaucoma, particularly primary open angle glaucoma (wide sense) or secondary open angle glaucoma in which the trabecular meshwork is involved, and ocular hypertension in which the trabecular meshwork is involved, based on the findings of the above-described novel action mechanism. Furthermore, it can be said that the present invention is a Rho kinase inhibitor-containing agent for improving and enhancing of aqueous humor outflow resistance, a Rho kinase inhibitor-containing agent for improving and enhancing of aqueous humor outflow resistance by long-term continuous administration, and a Rho kinase inhibitor-containing agent for adjusting trabecular meshwork function by long-term continuous administration.

In a case where the Rho kinase inhibitor of the present invention, for example, a preparation of (S)-(-)-1-(4-fluoro-5-isoquinolinesulfonyl)-2-methyl-1,4-homopiperazine, a salt thereof, or a solvate thereof is prepared, the preparation can be prepared according to a known method. For example, an ophthalmic agent can be prepared using an isotonizing agent, a buffer, a surfactant, or a preservative as necessary. The pH is only required to be within an allowable range for an ophthalmic preparation, and is preferably from pH 4 to 8.

The above preparation is preferably used for an ophthalmic preparation, particularly for ocular instillation. Such an ophthalmic agent can be any one of an aqueous ophthalmic agent, a non-aqueous ophthalmic agent, a suspension ophthalmic agent, an emulsion ophthalmic agent, and an eye ointment. Such a preparation can be manufactured by blending, as a composition suitable for an administration form, a pharmaceutically acceptable carrier, for example, an isotonizing agent, a chelating agent, a stabilizing agent, a pH adjusting agent, a preservative, an antioxidant, a dissolution aid, or a thickening agent as necessary according to a (preparation) method known to those skilled in the art.

An ophthalmic agent can be prepared, for example, by dissolving or suspending the above desired ingredient in sterilized purified water, an aqueous solvent such as physiological saline, or a nonaqueous solvent such as vegetable oil including cottonseed oil, soybean oil, sesame oil, and peanut oil, adjusting an osmotic pressure to a predetermined value, and performing a sterilization treatment such as filter sterilization. Note that an ointment base can be contained in addition to the above-described various ingredients in a case where an ophthalmic ointment is prepared. The above-described ointment base is not particularly limited, but examples thereof include an oily base such as petrolatum, liquid paraffin, or polyethylene; an emulsion base prepared by emulsifying an oil phase and an aqueous phase with a surfactant or the like; and a water-soluble base containing hydroxypropylmethyl cellulose, carboxymethyl cellulose, or polyethylene glycol.

In a case where a drug containing (S)-(-)-1-(4-fluoro-5-isoquinolinesulfonyl)-2-methyl-1,4-homopiperazine, a salt thereof, or a solvate thereof as the Rho kinase inhibitor of the present invention is used as the therapeutic agent of the present invention, a dosage thereof varies depending on body weight, age, sex, symptom, administration form, administration frequency, and the like of a patient. However, in general, (S)-(-)-1-(4-fluoro-5-isoquinolinesulfonyl)-2-methyl-1,4-homopiperazine, a salt thereof, or a solvate thereof is administered to an adult in an amount of 0.025 to 10000 µg, preferably 0.025 to 2000 µg, more preferably 0.1 to 2000 µg per day. In a case where another Rho kinase inhibitor is used as the therapeutic agent of the present invention, a dosage thereof can be appropriately adjusted in accordance with the above-described effective amount or dose depending on body weight, age, sex, symptom, administration form, administration frequency, and the like of a patient.

The number of times of administration is not particularly limited. However, it is preferable to be administered once or divided into several times. In a case of a liquid ophthalmic agent, it is only required to drop one to several drops into an eye at a time.

Hereinafter, the present invention will be described in more detail, but the present invention is not limited thereto.

### Examples

### Example 1

### Enhanced intra-ocular pressure-lowering effect caused by continuous ocular instillation of 0.4% ripasudil ophthalmic solution

### 1. Target and method

A multi-center open-label study was performed using a cohort for a monotherapy of ripasudil ophthalmic solution (173 cases) and a cohort for an additive therapy of a ripasudil ophthalmic solution in combination with another agent (181 cases) (additive therapy to a PG related drug: 62 cases, additive therapy to a β blocker: 60 cases, additive therapy to a combination drug of said PG-related drug and said β blocker: 59 cases). Patients with primary open angle glaucoma, ocular hypertension, and exfoliation glaucoma (one type of secondary glaucoma to cause aqueous humor outflow resistance due to exfoliation substances in aqueous humor or the like) having a pre-treatment intra-ocular pressure of 15 mmHg or higher or a post-treatment intra-ocular pressure of 15 mmHg or higher at the time after treatment of a PG-related drug, a β-blocker, or a combination drug of the PG-related drug and β-blocker were enrolled. A 0.4% ripasudil ophthalmic solution was dropped into an eye twice daily for 52 weeks. In order to evaluate intra-ocular pressure-lowering effect over time caused by long-term ocular instillation, the amount of change in intra-ocular pressure (least mean square value) in a second half of ocular instillation (32 weeks to 52 weeks) with respect to a first half of ocular instillation (4 weeks to 28 weeks) was examined. Statistical analysis was performed using SAS (SAS Inc., Cary, North Carolina) by a least squares method based on a mixed effect model.

### 2. Result

Results are illustrated in Table 1 and FIGS. 1 and 2.

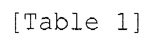

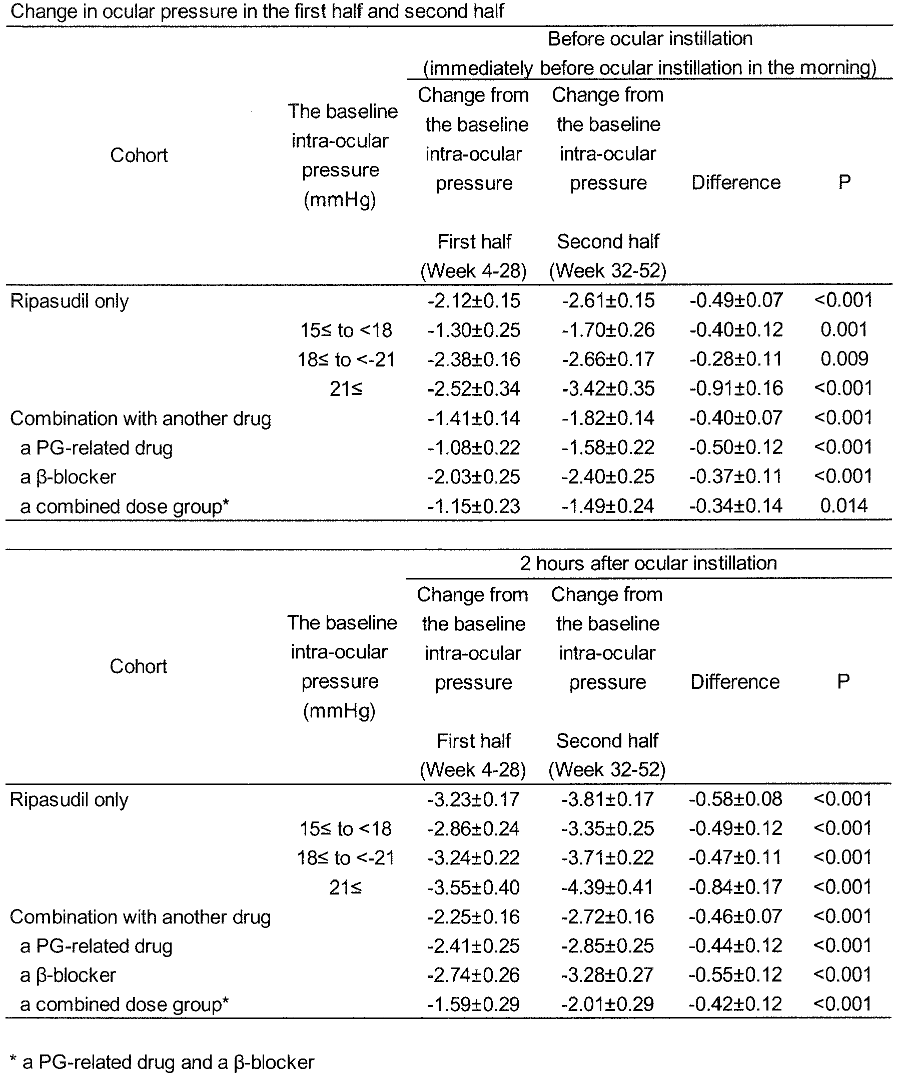

Table 1 and FIG. 1 indicate that a significant intra-ocular pressure-lowering effect was observed throughout an ocular instillation period in a cohort for monotherapy of ripasudil and a cohort for additive therapy of ripasudil and another agent (3 subcohortss) (32 weeks to 52 weeks, immediately before ocular instillation in the morning: - 2.61 mmHg and -1.82 mmHg, 32 weeks to 52 weeks, 2 hours after ocular instillation: -3.81 mmHg and -2.72 mmHg). As for the amount of change in intra-ocular pressure in a second half (32 weeks to 52 weeks) with respect to a first half (4 weeks to 18 weeks), immediately before ocular instillation in the morning, the intra-ocular pressure was -0.49 mmHg (p < 0.001) in the monotherapy group and -0.40 mmHg (p < 0.001) in the additive therapy group, and 2 hours after ocular instillation, the intra-ocular pressure was - 0.58 mmHg (p < 0.001) in the monotherapy group and -0.46 mmHg (p < 0.001) in the additive therapy group. The intra-ocular pressure was lowered in both groups, but was remarkably lowered in the monotherapy group. In the monotherapy group, this lowering effect showed a tendency of being larger in a subgroup having higher pre-treatment intra-ocular pressure (baseline intra-ocular pressure) (Table 1 and FIG. 2, a cohort with a baseline intra-ocular pressure of 21 mmHg or higher: -0.91 mmHg, p < 0.001). A similar tendency was observed in the intra-ocular pressure at 2 hours after ocular instillation.

Ripasudil exhibited a further intra-ocular pressure-lowering effect by continuous ocular instillation in addition to an intra-ocular pressure-lowering effect in an initial stage of ocular instillation. In addition, a similar effect was exhibited in combination with another drug.

### Industrial Applicability

The present invention can provide a drug for preventing or treating glaucoma or ocular hypertension, the drug exhibiting no decrease in efficacy during long-term administration and exhibiting an enhanced intra-ocular pressure-lowering effect, and therefore has industrial applicability.

## Claims

1. A therapeutic agent for glaucoma or ocular hypertension for long-term continuous administration, comprising a Rho kinase inhibitor.

2. The therapeutic agent according to claim 1, wherein the long-term continuous administration is 26 weeks or more.

3. An agent for improving and enhancing aqueous humor outflow resistance, comprising a Rho kinase inhibitor.

4. An agent for improving and enhancing aqueous humor outflow resistance for long-term continuous administration, comprising a Rho kinase inhibitor.

5. The agent for improving and enhancing aqueous humor outflow resistance according to claim 4, wherein the long-term continuous administration is 26 weeks or more.

6. A trabecular meshwork function adjusting agent for long-term continuous administration, comprising a Rho kinase inhibitor.

7. The trabecular meshwork function adjusting agent according to claim 6, wherein the long-term continuous administration is 26 weeks or more.

8. A therapeutic agent for glaucoma or ocular hypertension, comprising a Rho kinase inhibitor as an active ingredient, wherein the therapeutic agent is for use in the treatment of glaucoma or ocular hypertension in a patient who has been administered with a Rho kinase inhibitor-containing therapeutic agent for glaucoma or ocular hypertension over a definite period and is suffering from glaucoma or ocular hypertension considered to require a further enhanced intra-ocular pressure-lowering action.

9. The therapeutic agent according to claim 8, wherein the definite period for administration is 26 weeks or more.

10. The therapeutic agent according to claim 8 or 9, wherein the glaucoma or ocular hypertension considered to require a further enhanced intra-ocular pressure-lowering action is a glaucoma or ocular hypertension with a baseline intra-ocular pressure of 15 mmHg or higher at the time before treatment.
